# EUROPEAN PATENT SPECIFICATION

(11) **EP 3 868 743 B1**
(45) Date of publication and mention of the grant of the patent: **03.05.2023**
(21) Application number: 19872236.5
(22) Date of filing: 30.08.2019
(51) Int. Cl.: C07D 301/32, C07D 303/04, B01D 3/00, B01D 3/14, B01D 3/40

(54) **PROPYLENE OXIDE PURIFICATION SYSTEM AND METHOD FOR PRODUCING PROPYLENE OXIDE**
PROPYLENOXIDREINIGUNGSSYSTEM UND VERFAHREN ZUR HERSTELLUNG VON PROPYLENOXID
SYSTÈME DE PURIFICATION D'OXYDE DE PROPYLÈNE ET PROCÉDÉ DE PRODUCTION D'OXYDE DE PROPYLÈNE

(30) Priority: 11.10.2018 JP 2018192452
(43) Date of publication of application: 25.08.2021
(73) Proprietor: SUMITOMO CHEMICAL COMPANY LIMITED, Chuo-ku Tokyo 104-8260 (JP)
(72) Inventor: TAKEMOTO Takuo, Ichihara-shi, Chiba 2990195 (JP); NAKAMURA Motoshi, Ichihara-shi, Chiba 299-0195 (JP)
(74) Representative: Winter, Brandl - Partnerschaft mbB
(86) International application number: PCT/JP2019/034247
(87) International publication number: WO 2020/075417

(56) References cited:
- WO-A1-01/83468
- CN-A- 104 109 138
- JP-A- H07 309 853
- JP-A- 2005 089 413
- JP-A- 2005 281 163
- JP-A- 2006 232 744
- JP-B1- S5 319 562
- US-A- 3 881 996
- US-A- 5 106 458

## Description

### TECHNICAL FIELD

The present invention relates to a propylene oxide purification system, and a method for producing propylene oxide.

### BACKGROUND

Systems for purification of crude propylene oxide have been conventionally known. For example, Patent Literature 1 discloses a purification system using four distillation columns.

In the first distillation column, crude propylene oxide is distilled, to discharge high-boiling-point components having boiling points higher than that of propylene oxide from the column bottom and a propylene oxide-containing flow removed of the high-boiling-point components from the column top. In the second distillation column, the column top flow from the first distillation column is distilled together with an extractant, to discharge water from the column top and discharge a mixture of the extractant and propylene oxide removed of water from the column bottom. In the third distillation column, the mixture of the extractant and propylene oxide is distilled, to discharge the extractant from the column bottom and discharge purified propylene oxide from the column top. In the fourth distillation column, the extractant discharged from the column bottom of the third distillation column is distilled, and the extractant purified is recycled to the second distillation column.

### Citation List

### Patent Literature

Patent Literature 1: Chinese Patent Publication No. 104109138
Patent Literature 2: US 3 881 996 A discloses a process for recovering propylene oxide by distillation.

### SUMMARY

Conventional systems are, however, incapable of sufficiently removing C1-C4 hydrocarbons without loss of propylene oxide.

According to examination made by the present inventors, the flow rate of the column top flow from the second distillation column must be increased when hydrocarbons having one to four carbon atoms (hereinafter, referred to as C1-C4 hydrocarbons) contained in crude propylene oxide are to be removed by using any conventional system. As described in Patent Literature 1, however, propylene oxide is contained in the column top flow from the second distillation column, and hence the recovery rate for propylene oxide in crude propylene oxide is disadvantageously lowered if the flow rate of the column top flow from the second distillation column is increased.

The present invention has been made in view of such a problem, and provides a propylene oxide purification system capable of sufficiently removing C1-C4 hydrocarbons in propylene oxide to be obtained even when loss of propylene oxide is kept low.

The propylene oxide purification system according to the present invention comprises:
a first distillation column comprising an inlet configured to receive crude propylene oxide, a column bottom outlet, and a column top outlet;
a second distillation column comprising an inlet, a reflux inlet, a column bottom outlet and a column top outlet;
liquefaction separation equipment configured to liquefy at least part of a flow fed from the column top outlet of the second distillation column, and discharge a gas phase from a gas phase outlet and a liquid phase from a liquid phase outlet;
a third distillation column comprising an inlet, a column bottom outlet and a column top outlet;
a line LA connecting the column top outlet of the first distillation column and the inlet of the second distillation column;
a line LC connecting the liquid phase outlet of the liquefaction separation equipment and the reflux inlet of the second distillation column;
a line LD connecting the column bottom outlet of the second distillation column and the inlet of the third distillation column; and
a line LE connecting a source of extractant supply and at least one selected from the group consisting of the line LA, the liquefaction separation equipment, the line LC, and the second distillation column.

According to the present invention, through distillation of crude propylene oxide in the first distillation column, high-boiling-point components having boiling points higher than that of propylene oxide can be discharged from the column bottom and a propylene oxide-containing flow (F1) with reduced amounts of the high-boiling-point components can be discharged from the column top. An extractant can be fed from a source of extractant supply to at least one selected from the group consisting of the line LA, the liquefaction separation equipment, the line LC, and the second distillation column via the line LE; further, through distillation of the flow (F1) together with the extractant in the second distillation column, a flow (F2) comprising a C1-C4 hydrocarbon and propylene oxide can be discharged from the column top and a flow (F3) comprising the extractant and propylene oxide can be discharged from the column bottom. At this time, half or more of the total weight of propylene oxide in the flow (F1) is contained in the flow (F3). Through distillation of the flow (F3) in the third distillation column, a flow (FS) comprising the extractant can be discharged from the column bottom and purified propylene oxide can be discharged from the column top. At least part of the flow (F2) can be liquefied in the liquefaction separation equipment to separate a flow comprising a liquid phase and a gas phase into a liquid phase flow (F2O) and a gas phase flow (F2G), and the liquid phase flow (F2O) can be returned to the second distillation column via the liquid phase outlet and the line LC while the gas phase flow (F2G) can be discharged from the liquefaction separation equipment via the gas phase outlet without being returned to the second distillation column.

Thereby, high-boiling-point components can be removed from crude propylene oxide to less amounts in the first distillation column, C1-C4 hydrocarbons can be removed in the second distillation column and the liquefaction separation equipment, and propylene oxide of high purity can be obtained from the column top of the third distillation column.

In particular, loss of propylene oxide can be reduced with C1-C4 hydrocarbons removed because at least part of propylene oxide contained in the flow (F2) can be liquefied to be separated from a gas phase in the liquefaction separation equipment and returned to the second distillation column. C1-C4 hydrocarbons in the third distillation column are distributed to the column top together with propylene oxide, and hence if C1-C4 hydrocarbons are fed to the third distillation column, it is difficult to separate the C1-C4 hydrocarbons from propylene oxide. According to the present invention, however, the amounts of C1-C4 hydrocarbons in propylene oxide to be obtained from the third distillation column can be reduced by separating a gas comprising C1-C4 hydrocarbons from propylene oxide in the liquefaction separation equipment, providing propylene oxide with higher purity.

The liquefaction separation equipment can comprise: a gas-liquid separator comprising the gas phase outlet, the liquid phase outlet and a fluid inlet; a line LB connecting the column top outlet of the second distillation column and the fluid inlet of the gas-liquid separator; and a liquefaction section provided to the line LB or the gas-liquid separator.

The liquefaction section can comprise a condenser.

The liquefaction section can comprise a junction connecting the line LE and the line LB or the gas-liquid separator.

The liquefaction section can comprise a junction connecting a line LH connected to a source of water supply and the line LB or the gas-liquid separator, and can satisfy (a) or (b):
(a) the gas-liquid separator is a three-phase separator further comprising an aqueous phase outlet;
(b) an oil-water separator comprising a mixture inlet, an oil phase outlet, and an aqueous phase outlet is provided to the line LC, the mixture inlet is connected to the liquid phase outlet of the gas-liquid separator, and the oil phase outlet is connected to the reflux inlet of the second distillation column.

Once water is fed to the line LB or the gas-liquid separator, propylene oxide contained in the flow (F2) can be efficiently incorporated in the liquid phase (mixture of an aqueous phase and an oil phase) in the line LB or gas-liquid separator, and concentrations of water-soluble impurities contained in the liquid phase (mixture of an aqueous phase and an oil phase), such as formaldehyde, acetaldehyde, methanol, and acetone, can be reduced by separating the aqueous phase from the liquid phase with the three-phase separator or oil-water separator. Accordingly, the purity of propylene oxide to be obtained can be enhanced.

The liquefaction separation equipment in the system can further comprise: a fifth distillation column comprising an inlet, the gas phase outlet provided to a column top of the fifth distillation column, and the liquid phase outlet provided to a column bottom of the fifth distillation column; and a line LB connecting the inlet of the fifth distillation column and the column top outlet of the second distillation column.

The liquefaction separation equipment can further comprise a junction connecting the line LE and the line LB.

The liquefaction separation equipment can further comprise a condenser provided to the line LB.

In any of the systems, the system can further comprise a line LJ connecting a detoxification apparatus and the gas phase outlet of the liquefaction separation equipment.

In any of the systems, the source of extractant supply can be the column bottom outlet of the third distillation column.

In any of the systems, the system can further comprise:
a fourth distillation column comprising an inlet, a column bottom outlet and a column top outlet;
a line LF connecting the column bottom outlet of the third distillation column or a line connected to the column bottom outlet of the third distillation column and the inlet of the fourth distillation column; and
a line LG connecting the column bottom outlet of the fourth distillation column and at least one selected from the group consisting of the second distillation column, the liquefaction separation equipment, the line LC, the line LA, and the third distillation column.

The method for producing purified propylene oxide according to the present invention is a method for producing purified propylene oxide with use of the propylene oxide purification system in any of the above-described modes, comprising:
a step of distilling crude propylene oxide in the first distillation column, to discharge a high-boiling-point component having a boiling point higher than that of propylene oxide from a column bottom of the first distillation column and a propylene oxide-containing flow (F1) removed of the high-boiling-point component from a column top of the first distillation column;
a step of feeding an extractant to at least one selected from the group consisting of the line LA, the liquefaction separation equipment, the line LC, and the second distillation column;
a step of distilling the flow (F1) together with the extractant in the second distillation column, to discharge a flow (F2) comprising a C1-C4 hydrocarbon and propylene oxide from a column top of the second distillation column and a flow (F3) comprising the extractant and propylene oxide from a column bottom of the second distillation column;
a step of liquefying at least part of the propylene oxide contained in the flow (F2) in the liquefaction separation equipment, and discharging a gas phase flow (F2G) from the gas phase outlet and a liquid phase flow (F2O) from the liquid phase outlet;
a step of returning the liquid phase flow (F2O) to the second distillation column via the line LC; and
a step of distilling the flow (F3) in the third distillation column, to discharge a flow (FS) comprising the extractant from a column bottom of the third distillation column and purified propylene oxide from a column top of the third distillation column.

In this method, part of the flow (FS) of the extractant discharged from the column bottom outlet of the third distillation column can be fed to at least one selected from the group consisting of the line LA, the second distillation column, the liquefaction separation equipment, and the line LC via the line LE.

The method can comprise:
a step of distilling part of the flow (FS) of the extractant discharged from the column bottom of the third distillation column in the fourth distillation column, to discharge low-boiling-point components having boiling points lower than that of the extractant from a column top of the fourth distillation column and a flow (FSS) of the extractant removed of the low-boiling-point components from a column bottom of the fourth distillation column; and
a step of feeding the flow (FSS) of the extractant to at least one selected from the group consisting of the line LA, the second distillation column, the liquefaction separation equipment, the line LC, and the third distillation column via a line LG.

Provided according to the present invention is a propylene oxide purification system capable of reducing loss of propylene oxide with reduced concentrations of C1-C4 hydrocarbons in propylene oxide to be obtained, and a method with use of the propylene oxide purification system.

### BRIEF DESCRIPTION OF THE DRAWINGS

FIG. 1 shows a flowchart of a propylene oxide purification system according to a first embodiment of the present invention;
FIG. 2 shows a flowchart of a propylene oxide purification system according to a second embodiment of the present invention;
FIG. 3 shows a flowchart of a propylene oxide purification system according to a third embodiment of the present invention;
FIG. 4 shows a flowchart of a propylene oxide purification system according to a fourth embodiment of the present invention;
FIG. 5 shows a flowchart of a propylene oxide purification system according to a fifth embodiment of the present invention;
FIG. 6 shows a flowchart of a propylene oxide purification system according to a sixth embodiment of the present invention; and
FIG. 7 shows a flowchart of a propylene oxide purification system according to a seventh embodiment of the present invention.

### DETAILED DESCRIPTION

Systems and methods according to embodiments of the present invention will be described with reference to the drawings.

### (First embodiment)

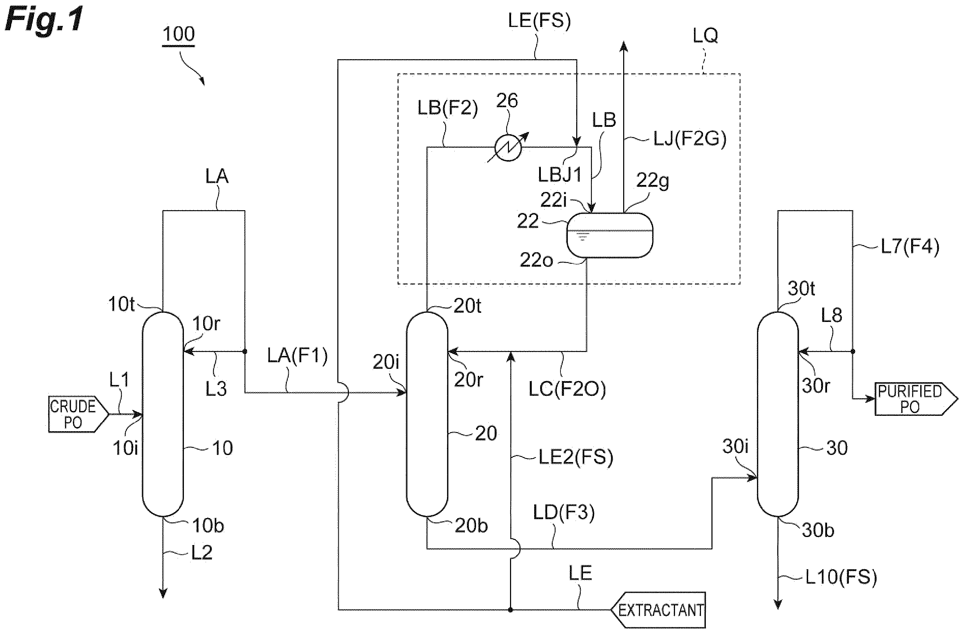

A propylene oxide purification system 100 according to the first embodiment will be described with reference to FIG. 1.

The propylene oxide purification system 100 primarily comprises: a first distillation column 10, a second distillation column 20, liquefaction separation equipment LQ, and a third distillation column 30.

The first distillation column 10 comprises an inlet 10i configured to be fed crude propylene oxide, in other words, configured to receive crude propylene oxide, a reflux inlet 10r, a column bottom outlet 10b, and a column top outlet 10t. The inlet 10i and a source of crude propylene oxide supply are connected together with a line L1. A line LA is connected to the column top outlet 10t of the first distillation column 10.

The second distillation column 20 comprises an inlet 20i, a reflux inlet 20r, a column bottom outlet 20b and a column top outlet 20t.

The column top outlet 10t of the first distillation column 10 and the inlet 20i of the second distillation column 20 are connected together with the line LA. A line L3 branching from the line LA is connected to the reflux inlet 10r of the first distillation column 10. The reflux inlet 10r is provided higher than the inlet 10i. On the upstream side than the branching point to the line L3 in the line LA (the column top outlet 10t side of the first distillation column 10), one or more condensers configured to convert at least part of a flow into a liquid phase and a gas-liquid separator may be provided in the order from the upstream side.

The liquefaction separation equipment LQ comprises a gas-liquid separator 22, a line LB, a condenser (liquefaction section) 26 and a junction (liquefaction section) LBJ1. The gas-liquid separator 22 comprises a fluid inlet 22i, a gas phase outlet 22g and a liquid phase outlet (oil phase outlet) 22o. The line LB connects the column top outlet 20t of the second distillation column 20 and the fluid inlet 22i of the gas-liquid separator 22. The condenser 26 is provided to the line LB. The junction LBJ1 is a part where the line LE and the line LB join together. In the present embodiment, the junction LBJ1 is provided to the line LB in the downstream side (gas-liquid separator 22 side) than the condenser 26. The line LE is connected to a source of extractant supply.

The condenser 26 and the junction LBJ1 each liquefy at least part of a flow (F2) fed from the column top outlet 20t of the second distillation column 20 via the line LB through phase change from gas to liquid by cooling and through contact between the flow (F2) and a liquid. The gas-liquid separator 22 subjects a fluid comprising a liquid and a gas fed from the fluid inlet 22i to gas-liquid separation, and discharges the gas phase from the gas phase outlet 22g and discharges the liquid phase from the liquid phase outlet (oil phase outlet) 22o. The condenser 26 can be a heat exchanger to which a refrigerant is fed. The shape of the heat exchanger is not limited, and examples thereof comprise shell and tube heat exchangers and plate heat exchangers. An example of operation conditions for the condenser 26 is such that cooling is performed so that the exit temperature of the flow (F2) reaches equal to or lower than the boiling point of propylene oxide, or so that at least a liquid phase is generated at the outlet for the flow (F2).

For the specific configuration of the gas-liquid separator 22, various forms of configurations can be used such that a fluid comprising a liquid and a gas is reserved to form a gas-liquid interface separating the gas phase above the gas-liquid interface and the liquid phase below the gas-liquid interface, and that the gas phase and the liquid phase can be independently discharged from the gas phase outlet and the liquid phase outlet, respectively. Typically, the liquid phase outlet 22o is provided in the bottom of the gas-liquid separator 22 of drum shape, and the gas phase outlet 22g is provided in an upper part of the gas-liquid separator 22.

The liquid phase outlet 22o of the gas-liquid separator 22 is connected to the reflux inlet 20r of the second distillation column 20 via a line LC. The reflux inlet 20r is provided higher than the inlet 20i. A line LJ is connected to the gas phase outlet 22g of the gas-liquid separator 22. A detoxification apparatus configured to incinerate compounds in the gas phase may be connected to the line LJ. A line LE2 joins to the line LC. The line LE2 is connected to the source of extractant supply via the line LE.

The third distillation column 30 comprises a first inlet 30i, a reflux inlet 30r, a column bottom outlet 30b and a column top outlet 30t. The reflux inlet 30r is provided higher than the first inlet 30i.

The column bottom outlet 20b of the second distillation column 20 and the first inlet 30i of the third distillation column 30 are connected together with a line LD.

A line L7 is connected to the column top outlet 30t of the third distillation column 30. A line L8 branches from the line L7, and the line L8 is connected to the reflux inlet 30r. On the upstream side than the branching point to the line L8 in the line L7 (the column top outlet 30t side of the third distillation column 30), one or more condensers configured to convert at least part of a flow into a liquid phase and a gas-liquid separator may be provided in the order from the upstream side. A line L10 is connected to the column bottom outlet 30b of the third distillation column 30.

### (Method for producing propylene oxide)

Subsequently, a method for producing propylene oxide with use of the propylene oxide purification system 100 according to the first embodiment will be described.

First, crude propylene oxide is fed from the source of crude propylene oxide supply to the first distillation column 10 via the line L1.

### (Crude propylene oxide)

The crude propylene oxide comprises impurities in addition to propylene oxide. Examples of such impurities can comprise organic acids such as formic acid, acetic acid and propionic acid; esters such as methyl formate; water; alcohols such as methanol, ethanol, n-propyl alcohol, and isopropyl alcohol; glycols such as propylene glycol; ketones such as acetone; aldehydes such as formaldehyde, acetaldehyde, and propionaldehyde; hydrocarbons having one to four carbon atoms (referred to as C1-C4 hydrocarbons) such as methane, ethane, propane, propylene, cyclopropane, n-butane, isobutane, 1-butene, 2-butene, and butadiene; pentanes, pentens, pentadienes, hexanes, hexenes, and hydrocarbons comprising hexadienes (referred to as C5-C6 hydrocarbons).

Examples of the crude propylene oxide comprise a composition obtained by reacting a peroxide and propylene in the presence of a catalyst. A part of impurities can be removed in advance from the composition, as necessary.

Examples of the peroxide comprise hydrogen peroxide, tert-butylbenzene hydroperoxide, ethylbenzene hydroperoxide, and cumene hydroperoxide. Examples of the catalyst for reaction between the peroxide and propylene comprise catalysts comprising titanium chemically bonding to a silicon oxide, namely, what is called titanium-silica catalysts.

It is preferable to react the peroxide and propylene in a solvent. The solvent needs to be liquid at the temperature and pressure in the reaction and substantially inert to the reactants and products. Examples of the solvent comprise cumene, aromatic monocyclic compounds (e.g., benzene, toluene, chlorobenzene, orthodichlorobenzene), alkanes (e.g., octane, decane, dodecane), alcohols (methanol, ethanol), and water. The reaction temperature is typically 0 to 200°C, and a temperature of 25 to 200°C is preferred. The pressure needs to be a pressure enough to keep the reaction mixture in a liquid state. It is generally advantageous that the pressure be 100 to 10000 kPa.

In the first distillation column 10, the crude propylene oxide is distilled, to discharge high-boiling-point components having boiling points higher than that of propylene oxide from the column bottom outlet 10b and a propylene oxide-containing flow (F1) removed of the high-boiling-point components from the column top outlet 10t to be fed to the second distillation column 20.

Examples of impurities having boiling points higher than that of propylene oxide comprise organic acids such as formic acid, acetic acid, and propionic acid; esters such as methyl formate; water; alcohols such as methanol, ethanol, n-propyl alcohol, and isopropyl alcohol; glycols such as propylene glycol; ketones such as acetone; aldehydes such as formaldehyde, acetaldehyde, and propionaldehyde; hydrocarbons having one to four carbon atoms (referred to as C1-C4 hydrocarbons) such as methane, ethane, propane, cyclopropane, propylene, n-butane, isobutane, 1-butene, 2-butene, and butadiene; and hydrocarbons comprising pentanes, pentenes, pentadienes, hexanes, hexenes and hexadienes (referred to as C5-C6 hydrocarbons).

An example of preferred operation conditions for the first distillation column 10 is as follows: the number of theoretical plates is 5 to 200, the operation pressure is 0.01 to 5 MPa in absolute pressure, and the temperature is between 0°C and 300°C.

The degree of removal of the high-boiling-point components can be controlled through refluxing part of the flow (F1) discharged from the column top outlet 10t to the first distillation column 10 via the line L3.

Subsequently, the flow (F1) is fed to the second distillation column 20 via the line LA, and distilled. In the second distillation column 20, distillation is performed in the presence of an extractant fed via the line LE, the line LB, the gas-liquid separator 22, and the line LC.

Examples of the extractant comprise saturated hydrocarbons having 7 to 10 carbon atoms such as heptane, octane, nonane, and decane; aromatic hydrocarbons such as toluene, ethylbenzene, n-propylbenzene, and isopropylbenzene; ketones such as acetone; and glycols such as ethylene glycol and propylene glycol. Among them, saturated hydrocarbons having 7 to 10 carbon atoms and aromatic hydrocarbons are preferred, and saturated hydrocarbons having 7 to 10 carbon atoms are the most preferred. Addition of such an extractant increases the relative difference in volatility between low-boiling-point components such as methanol, water, acetaldehyde, and hydrocarbons and propylene oxide to facilitate separation of them.

In the second distillation column 20, through distillation in the presence of the extractant, a flow (F2) comprising water, formaldehyde, acetaldehyde, methanol, methyl formate and so on is discharged from the column top outlet 20t and a flow (F3) comprising propylene oxide and the extractant is discharged from the column bottom outlet 20b. The propylene oxide concentration of the flow (F3) is higher than that of the flow (F1).

An example of preferred distillation conditions for the second distillation column is as follows: the number of theoretical plates is 5 to 200, the operation pressure is 0.01 to 5 MPa in absolute pressure, and the temperature is between 0°C and 300°C. The weight ratio of the amount of the extractant to be fed to the second distillation column to the weight of propylene oxide to be fed can be 0.1 to 20.

Next, the flow (F2) discharged from the column top outlet 20t is cooled with the condenser 26 provided to the line LB to liquefy part of the flow (F2). Specifically, it is preferable to lower the temperature of the flow (F2) to a temperature equal to or lower than the boiling point of propylene oxide to liquefy at least part of propylene oxide.

The flow (FS) in liquid of the extractant fed via the line LE is fed from the junction LBJ1 to the line LB, and part of gas in the flow (F2) is dissolved/absorbed in the liquid phase of the extractant, in other words, part of gas in the flow (F2) is liquefied. Specifically, it is preferable to liquefy part of propylene oxide gas in the flow (F2), in other words, allow the liquid phase to dissolve/absorb therein part of propylene oxide gas in the flow (F2).

In the gas-liquid separator 22, a mixture of the flow (F2) and the flow (FS) is separated into a gas phase and a liquid phase. Then, a flow (F2G) of the gas phase is discharged to the outside via the gas phase outlet 22g and the line LJ. This gas phase primarily comprises C1-C4 hydrocarbons. A detoxification apparatus configured to remove C1-C4 hydrocarbons can be connected to the line LJ, and detoxification can be performed, as necessary. Examples of the detoxification apparatus comprise adsorbers configured to adsorb hydrocarbons and combustion apparatuses configured to combust C1-C4 hydrocarbons. One or more detoxification apparatus configured to remove C1-C4 hydrocarbons can be connected to the line LJ.

A flow (F2O) of the liquid phase is fed to the reflux inlet 20r of the second distillation column 20 via the liquid phase outlet 22o and the line LC. The flow (FS) in liquid of the extractant fed via the line LE2 may be fed to the line LC, as necessary.

Operation conditions for the gas-liquid separator 22 need to be conditions allowing formation of an interface of a gas phase and a liquid phase. It is preferable that the temperature be 0 to 80°C and that the pressure be normal pressure to 2 MPa.

Subsequently, the flow (F3) discharged from the column bottom outlet 20b of the second distillation column 20 is fed to the first inlet 30i of the third distillation column 30 via the line LD.

In the third distillation column 30, the flow (F3) comprising the extractant and propylene oxide is distilled, to discharge a flow (F4) of purified propylene oxide from the column top outlet 30t via the line L7, and on the other hand discharge the flow (FS) of the extractant from the column bottom outlet 30b via the line L10. The flow (FS) can be fed to the line LB and/or the line LC, for example, via the line LE, as necessary. Needless to say, the extractant may not be recycled. The extractant can be further purified and recycled.

Part of the flow (F4) discharged from the column top outlet 30t can be refluxed to the reflux inlet 30r of the third distillation column 30 via the line L8 to control the purity of the extractant contained in the flow (F4). In the described manner, the flow (F4) of purified propylene oxide can be obtained from the line L7.

Preferred operation conditions for the third distillation column 30 is as follows: the number of theoretical plates is 5 to 200, the operation pressure is 0.01 to 5 MPa in absolute pressure, and the temperature is between 0°C and 300°C.

### (Operational advantages)

According to the present embodiment, through distillation of crude propylene oxide in the first distillation column 10, high-boiling-point components having boiling points higher than that of propylene oxide can be discharged from the column bottom outlet 10b and a propylene oxide-containing flow (F1) with reduced amounts of the high-boiling-point components can be discharged from the column top outlet 10t. In addition, through distillation of the flow (F1) together with an extractant in the second distillation column 20, a flow (F2) comprising C1-C4 hydrocarbons and propylene oxide can be discharged from the column top outlet 20t and a flow (F3) comprising the extractant and propylene oxide can be discharged from the column bottom. At this time, half or more of the total weight of propylene oxide in the flow (F1) is contained in the flow (F3). Through distillation of the flow (F3) in the third distillation column 30, a flow (FS) comprising the extractant can be discharged from the column bottom outlet 30b and purified propylene oxide can be discharged as a flow (F4) from the column top outlet 30t.

In the liquefaction separation equipment LQ, at least part of the flow (F2) is liquefied to separate the flow comprising a liquid phase and a gas phase into a liquid phase and a gas phase, and a flow (F2O) of the liquid phase is returned to the second distillation column 20 via the liquid phase outlet 22o and the line LC while a gas phase flow (F2G) can be discharged from the liquefaction separation equipment LQ via the gas phase outlet 22g without being returned to the second distillation column 20.

Thereby, high-boiling-point components can be removed from crude propylene oxide to less amounts in the first distillation column 10, C1-C4 hydrocarbons can be removed in the second distillation column 20 and the liquefaction separation equipment LQ, and propylene oxide of high purity can be obtained from the column top of the third distillation column 30.

In particular, loss of propylene oxide can be reduced with C1-C4 hydrocarbons removed, because at least part of propylene oxide contained in the flow (F2) is liquefied and separated from the gas phase in the liquefaction separation equipment LQ, and returned to the second distillation column 20. C1-C4 hydrocarbons in the third distillation column 30 are distributed to the column top outlet 30t together with propylene oxide, and hence if C1-C4 hydrocarbons are fed to the third distillation column 30, it is difficult to separate the C1-C4 hydrocarbons from propylene oxide. According to the present embodiment, however, the amounts of C1-C4 hydrocarbons in propylene oxide to be obtained from the third distillation column 30 can be reduced by separating a gas comprising C1-C4 hydrocarbons from propylene oxide in the liquefaction separation equipment LQ, raising the purity of propylene oxide.

### (Second embodiment)

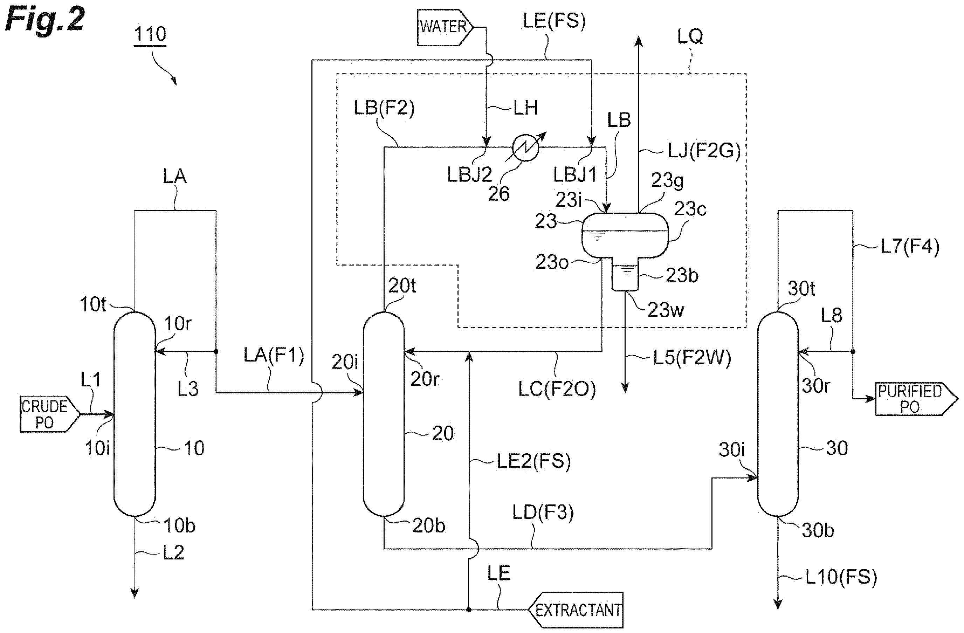

A propylene oxide purification system 110 according to the second embodiment will be described with reference to FIG. 2. In the following, only differences from the first embodiment will be described.

Differences of the present embodiment from the first embodiment is that a junction LBJ2 is provided which connects the line LB and the line LH connected to the source of water supply, and that a three-phase separator 23 is provided in place of the gas-liquid separator 22.

The junction LBJ2, which is provided to the line LB between the condenser 26 and the second distillation column 20 in the present embodiment, may be provided between the condenser 26 and the three-phase separator 23.

The line LH feeds water in liquid or gas from the junction LBJ2 to the line LB. The amount of water fed can be determined so that the ratio of the amount of water to the weight of the flow (F2) fed from the column top outlet 20t satisfies water/column top flow = 0.01/1 to 1/1.

The three-phase separator 23 comprises a fluid inlet 23i, a gas phase outlet 23g, an oil phase outlet (liquid phase outlet) 23o, and an aqueous phase outlet 23w, and the three-phase separator 23 separates a fluid into a gas phase, an aqueous phase and an oil phase. The fluid inlet 23i is connected to the line LB, the gas phase outlet 23g is connected to the line LJ, the oil phase outlet (liquid phase outlet) 23o is connected to a line LC, and the aqueous phase outlet 23w is connected to a line L5.

Specifically, the three-phase separator 23 can be a drum comprising a main drum 23c and a boot part 23b protruding downward from the main drum 23c, as illustrated in FIG. 2. Such a three-phase separator 23 can form an oil-water interface in the boot part 23b and a gas-liquid (gas-oil) interface in the main drum 23c. The fluid inlet 23i can be provided to an upper part of the main drum 23c, the oil phase outlet 23o can be provided to a lower part of the main drum 23c, and the aqueous phase outlet 23w can be provided to a lower part of the boot part 23b.

In the present embodiment, water in liquid or gas is fed from the line LH to the junction LBJ2 in the line LB. The junction LBJ2 allows at least part of the flow (F2) to be dissolved/absorbed in water by contacting the flow (F2) fed from the column top outlet 20t of the second distillation column 20 via the line LB with water to liquefy part of gas in the flow (F2). Specifically, part of propylene oxide gas and components soluble/absorbable in water (e.g., aldehydes such as formaldehyde and acetaldehyde, alcohols such as methanol, glycols such as propylene glycol, ketones such as acetone, esters such as methyl formate) can be liquefied by allowing them to be dissolved/absorbed in water.

Further, the three-phase separator 23 separates a mixture of the flow (F2), water, and the flow (FS) into a gas phase, an aqueous phase, and an oil phase. Then, a flow (F2G) of the gas phase is discharged to the outside via the line LJ, a flow (F2O) of the oil phase is returned to the reflux inlet 20r of the second distillation column 20 via the line LC, and a flow (F2W) of the aqueous phase is discharged to the outside via the line L5 without being returned to the second distillation column 20. A well-known water treatment apparatus can be connected to the line L5.

According to the present embodiment, at least part of the flow (F2) is liquefied by the condenser 26, and the junction LBJ2 and the junction LBJ1, and the fluid comprising a liquid and a gas is separated into a gas phase, an aqueous phase, and an oil phase by the three-phase separator 23. The oil phase is refluxed from the oil phase outlet (liquid phase outlet) 23o to the second distillation column 20 via the line LC, the aqueous phase is discharged from the aqueous phase outlet 23w via the line L5, and the gas phase is discharged from the gas phase outlet 23g in the same manner as in the first embodiment.

In the present embodiment, in particular, water-soluble/water-absorbable gas components in the flow (F2) are dissolved/absorbed in water through feeding water to the line LB by the junction LBJ2. Specifically, part of water-soluble/water-absorbable components in the flow (e.g., aldehydes such as formaldehyde and acetaldehyde, alcohols such as methanol, glycols such as propylene glycol, ketones such as acetone, esters such as methyl formate) are dissolved/absorbed in the aqueous phase. Then, the water-soluble/water-absorbable components can be separated through discharging the aqueous solution separated from the oil phase by the three-phase separator 23, without returning to the second distillation column 20, and thus the purity of purified propylene oxide can be raised.

### (Third embodiment)

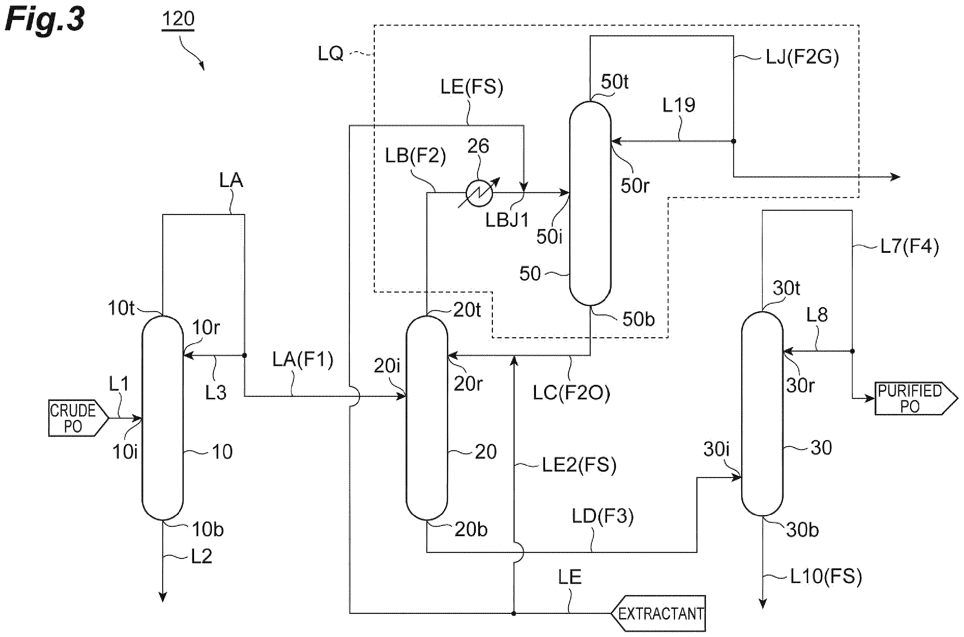

Subsequently, a propylene oxide purification system 120 according to the third embodiment of the present invention will be described with reference to FIG. 3. In the following, only a difference from the first embodiment will be described.

A difference of the present embodiment from the first embodiment is in the configuration of the liquefaction separation equipment LQ. The liquefaction separation equipment LQ in the present embodiment primarily comprises a fifth distillation column (liquefaction section) 50 and a line LB.

The fifth distillation column 50 comprises an inlet 50i, a reflux inlet 50r, a column top outlet (gas phase outlet) 50t and a column bottom outlet (liquid phase outlet) 50b. The line LB connects the column top outlet 20t of the second distillation column 20 and the inlet 50i of the fifth distillation column 50. To the line LB, similarly to the first embodiment, a junction LBJ1 to the line LE and a condenser 26 are provided. Although it is preferable that the condenser 26 be provided between the junction LBJ1 and the second distillation column 20, the condenser 26 may be provided between the junction LBJ1 and the fifth distillation column 50.

A line LJ is connected to the column top outlet 50t of the fifth distillation column 50. A line L19 branches from the line LJ, and the line L19 is connected to the reflux inlet 50r. The reflux inlet 50r is provided higher than the inlet 50i. On the upstream side than the branching point to the line L19 in the line LJ (the column top outlet 50t side of the fifth distillation column 50), a condenser configured to convert at least part of a flow into a liquid phase and a gas-liquid separator may be provided in the order from the upstream side.

The column bottom outlet 50b of the fifth distillation column 50 is connected to the reflux inlet 20r of the second distillation column 20 via the line LC.

In the present embodiment, distillation is performed in the first distillation column 10, the second distillation column 20, and the third distillation column 30 in the same manner as in the first embodiment, and distillation is additionally performed in the fifth distillation column 50. Specifically, a mixture of the flow (F2) that is discharged from the column top outlet 20t of the second distillation column 20 and comprises C1-C4 hydrocarbons and propylene oxide and the flow (FS) of the extractant fed from the line LE is distilled in the fifth distillation column 50, to discharge a flow (F2G) primarily comprising C1-C4 hydrocarbons via the column top outlet 50t and the line LJ and discharge a flow (F2O) comprising the extractant and propylene oxide with reduced amounts of C1-C4 hydrocarbons from the column bottom outlet 50b to the reflux inlet 20r of the second distillation column 20.

An example of preferred operation conditions for the fifth distillation column 50 is temperature and pressure conditions such that C1-C4 hydrocarbons in a fluid flowing from the inlet 50i can be extracted to the column top outlet 50t and propylene oxide and also that the extractant therein can be extracted to the column bottom outlet 50b. Specifically, the number of theoretical plates is 5 to 200, the operation pressure is 0.01 to 5 MPa in absolute pressure, and the temperature is between 0°C and 300°C.

Thereby, propylene oxide in the flow (F2) fed from the column top outlet 20t of the second distillation column 20 is liquefied in the fifth distillation column 50, and discharged as a liquid from the column bottom outlet 50b corresponding to a liquid phase outlet, and on the other hand C1-C4 hydrocarbons in the flow (F2) remain as gas, and discharged from the column top outlet 50t corresponding to a gas phase outlet.

In the present embodiment, part of the flow (F2) discharged from the column top outlet 20t of the second distillation column 20 can be liquefied by cooling with the condenser 26 provided to the line LB in the same manner as in the first embodiment. In addition, part of a gas in the flow (F2) can be liquefied by feeding the flow (FS) in liquid of the extractant fed via the line LE from the junction LBJ1 to the line LB.

According to the present embodiment, at least part of propylene oxide contained in the flow (F2) is liquefied by the liquefaction separation equipment LQ to be separated from the gas phase and returned to the second distillation column 20 in the same manner as in the first embodiment, and hence loss of propylene oxide can be reduced with C1-C4 hydrocarbons removed.

### (Fourth embodiment)

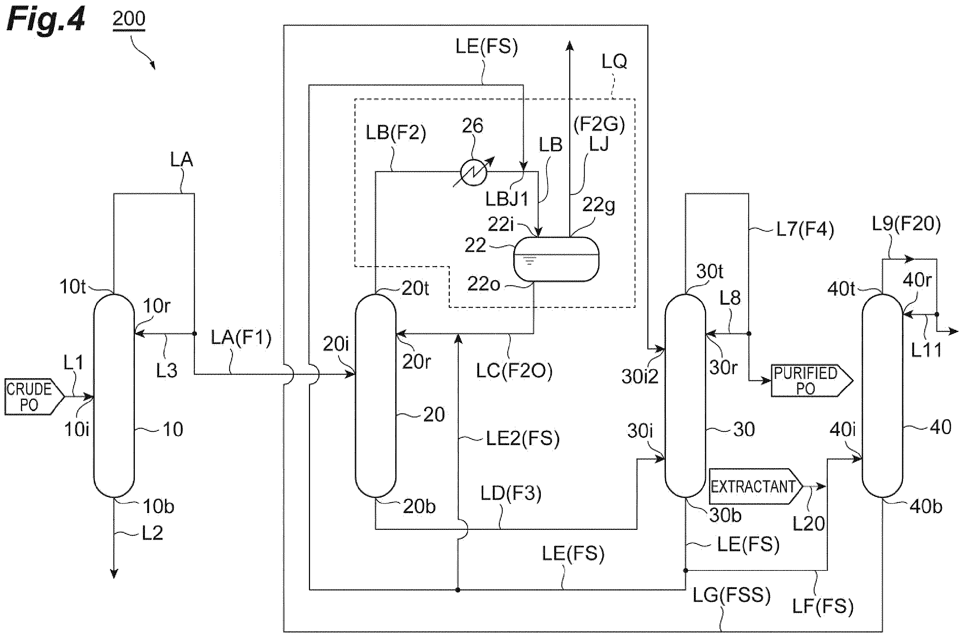

Subsequently, a propylene oxide purification system 200 according to the fourth embodiment of the present invention will be described with reference to FIG. 4. In the following, only differences from the first embodiment will be described.

Primary differences of the present embodiment from the first embodiment are that the source of extractant supply for the lines LE and LE2 is the column bottom outlet 30b of the third distillation column 30, and that a fourth distillation column 40, a line LF, and a line LG are included.

The column bottom outlet 30b of the third distillation column 30 and the line LE are connected together, and the flow (FS) of the extractant discharged from the column bottom outlet 30b of the third distillation column 30 is fed to the line LE and the line LE2, as described above.

The fourth distillation column 40 comprises an inlet 40i, a reflux inlet 40r, a column bottom outlet 40b and a column top outlet 40t. The line LE and the inlet 40i of the fourth distillation column 40 are connected together via the line LF. A line L20 connected to the source of extractant supply joins to the line LF.

A line L9 is connected to the column top outlet 40t of the fourth distillation column 40. A line L11 branches from the line L9, and the line L11 is connected to the reflux inlet 40r. The reflux inlet 40r is provided higher than the inlet 40i. On the upstream side than the branching point to the line L11 in the line L9 (the column top outlet 40t side of the fourth distillation column 40), a condenser configured to convert at least part of a flow into a liquid phase and a gas-liquid separator may be provided in the order from the upstream side.

The third distillation column 30 comprises a second inlet 30i2 positioned higher than the first inlet 30i, and the column bottom outlet 40b of the fourth distillation column 40 is connected to the second inlet 30i2 of the third distillation column 30 with the line LG. The second inlet 30i2 is positioned lower than the reflux inlet 30r.

In the present embodiment, part of a flow (FS) of the extractant discharged from the column bottom outlet 30b of the third distillation column 30 is distilled in the fourth distillation column 40, to discharge a flow (F20) primarily comprising components having boiling points lower than that of the extractant from the column top outlet 40t and the line L9 and discharge a flow (FSS) having higher purity than the flow (FS) from the column bottom outlet 40b. In the flow (FS) of the extractant discharged from the column bottom outlet 30b, at least a part of components having boiling points higher than that of propylene oxide (e.g., hydrocarbons comprising pentanes, pentenes, pentadienes, hexanes, hexenes and hexadienes (referred to as C5-C6 hydrocarbons)) are often contained. The flow (FS) can be removed of the above-described components to less concentrations by the fourth distillation column 40 to obtain the flow (FSS) of high purity.

An example of preferred operation conditions for the fourth distillation column 40 is as follows: the number of theoretical plates is 5 to 200, the operation pressure is 0.01 to 5 MPa in absolute pressure, and the temperature is between 0°C and 300°C.

The purity of the extractant contained in the flow (FSS) can be controlled by refluxing part of the flow (F20) discharged from the column top outlet 40t to the reflux inlet 40r of the fourth distillation column 40 via the line L11.

Thereby, the flow (FSS) of the extractant having higher purity than the flow (FS) of the extractant, which is discharged from the column bottom outlet 30b of the third distillation column 30, is fed from the column bottom outlet 40b of the fourth distillation column 40 to the second inlet 30i2 of the third distillation column 30 via the line LG.

Because such a flow (FSS) of the extractant of high purity is fed to a higher stage than the first inlet 30i for the flow (F3), extraction distillation occurs also in the third distillation column 30, and residual impurities (e.g., C5-C6 hydrocarbons) are distributed to the column bottom side, more specifically, to the flow (FS) via the column bottom outlet 30b, furthermore raising the purity of propylene oxide advantageously.

In addition, the line LE and the column bottom outlet 30b of the third distillation column 30 are connected together, and hence extraction distillation in the second distillation column 20 can be preferably performed through recycling the extractant.

### (Fifth embodiment)

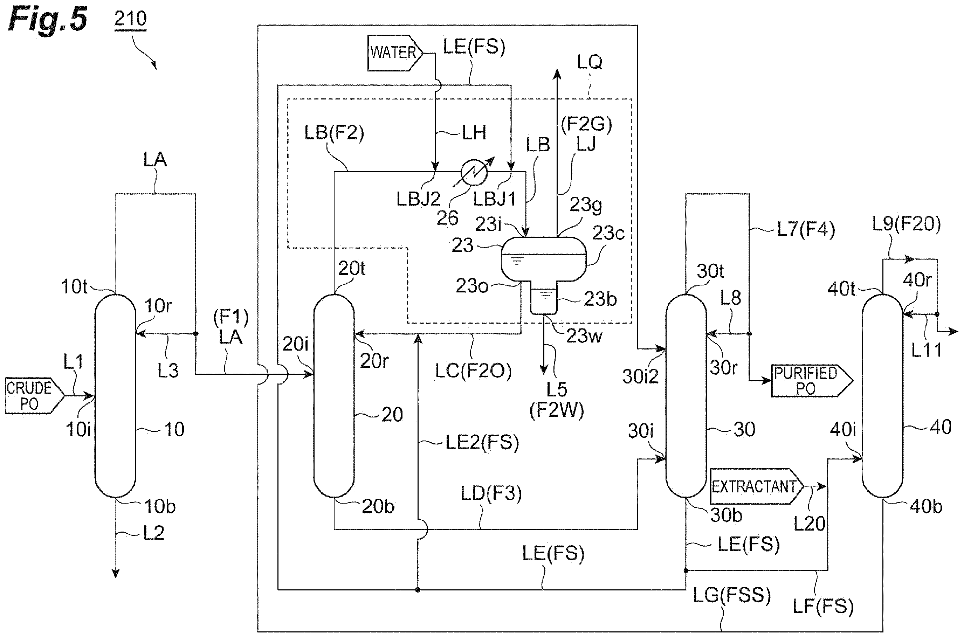

Subsequently, a propylene oxide purification system 210 according to the fifth embodiment of the present invention will be described with reference to FIG. 5. In the following, only differences from the second embodiment will be described.

Primary differences of the present embodiment from the second embodiment are that the source of extractant supply for the lines LE and LE2 is the column bottom outlet 30b of the third distillation column 30, and that a fourth distillation column 40, a line LF, and a line LG are included.

Specifically, details of these two points are the same as for the fourth embodiment, and thus description will be omitted.

### (Sixth embodiment)

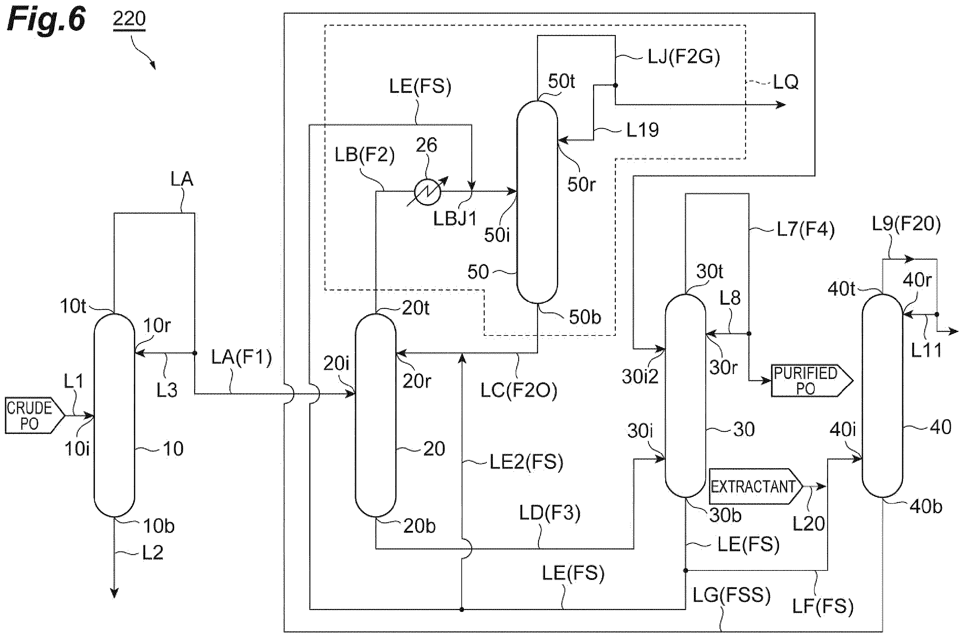

Subsequently, a propylene oxide purification system 220 according to the sixth embodiment of the present invention will be described with reference to FIG. 6. In the following, only differences from the third embodiment will be described.

Primary differences of the present embodiment from the third embodiment are that the source of extractant supply for the lines LE and LE2 is the column bottom outlet 30b of the third distillation column 30, and that a fourth distillation column 40, a line LF, and a line LG are included.

Specifically, details of these two points are the same as for the fourth embodiment, and thus description will be omitted.

### (Seventh embodiment)

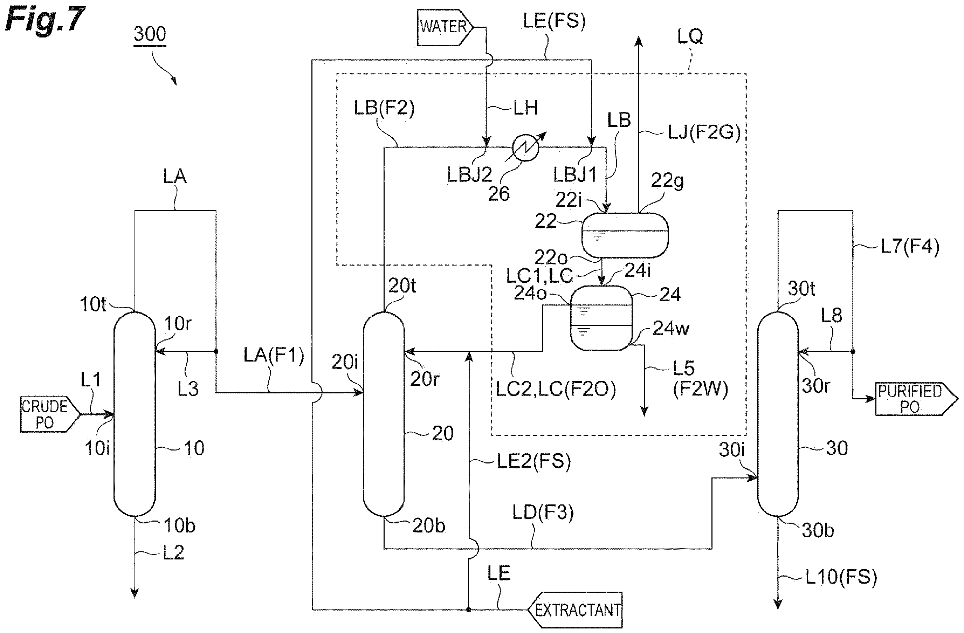

Subsequently, a propylene oxide purification system 300 according to the seventh embodiment of the present invention will be described with reference to FIG. 7. In the following, only a difference from the second embodiment will be described.

A primary difference of the present embodiment from the second embodiment is that combination of a gas-liquid separator 22 and an oil-water separator 24 is used in place of the three-phase separator 23. The gas-liquid separator 22 is the same as described for the first embodiment.

The oil-water separator 24 comprises a mixture inlet 24i configured to receive a liquid comprising oil and water, an oil phase outlet 24o, and an aqueous phase outlet 24w. The liquid phase outlet 22o of the gas-liquid separator 22 and the mixture inlet 24i of the oil-water separator 24 are connected together with a line LC1 (LC). The oil phase outlet 24o of the oil-water separator 24 and the reflux inlet 20r of the second distillation column 20 are connected together with a line LC2 (LC), and the line L5 is connected to the aqueous phase outlet 24w of the oil-water separator 24.

For the oil-water separator 24, various forms of configurations can be used such that a liquid comprising oil and water is reserved to form an oil-water interface separating the oil phase above the oil-water interface and the aqueous phase below the oil-water interface, and that the oil phase and the aqueous phase can be independently discharged from the oil phase outlet 24o and the aqueous phase outlet 24w, respectively. Typically, the oil phase outlet 24o is provided in the upper part of the oil-water separator 24 of drum shape, and the aqueous phase outlet 24w is provided in a bottom part of the oil-water separator 24.

According to the present embodiment, a liquid phase, that is, a mixture of an aqueous phase and an oil phase, and a gas phase are separated from each other in the gas-liquid separator 22, and a flow (F2G) of the gas phase is discharged via the gas phase outlet 22g and the line LF. A flow of the mixture of an aqueous phase and an oil phase is, on the other hand, fed to the oil-water separator 24 via the liquid phase outlet 22o, the line LC1, and the mixture inlet 24i to be separated into an oil phase and an aqueous phase, and a flow (F2O) of the oil phase is discharged via the oil phase outlet 24o and the line LC while a flow (F2W) of the aqueous phase is discharged via the aqueous phase outlet 24w and the line L5. Thereby, the same operational advantage as of the second embodiment is exerted.

The present invention is not limited to the above embodiments, and can be implemented in various modified modes.

### (Gas-liquid separator, three-phase separator, and oil-water separator)

For example, the forms of the gas-liquid separator 22, the three-phase separator 23, and the oil-water separator 24 are not limited to those described above. The gas-liquid separator 22 only needs to be able to separate a mixture of a gas phase and a liquid phase into a gas phase and a liquid phase, and discharge the gas phase and the liquid phase separately from each other. The three-phase separator 23 only needs to be able to separate a mixture of a gas phase, an aqueous phase, and an oil phase into a gas phase, an aqueous phase, and an oil phase, and discharge the gas phase, the aqueous phase, and the oil phase separately from each other. The oil-water separator 24 only needs to be able to separate a mixture of an oil phase and an aqueous phase into an oil phase and an aqueous phase, and discharge the oil phase and the aqueous phase separately from each other. The shape of each separator is not limited, and if having drum shape, for example, the separator may be of vertical type or of horizontal type. A baffle for gas-liquid separation or oil-water separation may be included in the separator. For example, in the system 210 in FIG. 5, combination of a gas-liquid separator 22 and an oil-water separator 24 as in the seventh embodiment may be employed in place of the three-phase separator 23.

### (Liquefaction section of liquefaction separation equipment LQ)

Although the line LB of the liquefaction separation equipment LQ in the first and fourth embodiments comprises both the junction LBJ1 and the condenser 26 as a liquefaction section, the line LB may comprise only one of them. The line LB may comprise two or more condensers. Of the junction LBJ1 and the condenser 26, a member not provided to the line LB can be provided to the other member of the liquefaction separation equipment LQ, that is, the gas-liquid separator 22. The first and fourth embodiments are implementable even in the case that the line LB of the liquefaction separation equipment LQ comprises none of the junction LBJ1 and the condenser 26 but that the junction LBJ1 and/or the condenser 26 are/is provided to the other member of the liquefaction separation equipment LQ, that is, the gas-liquid separator 22. In the case that the junction LBJ1 is not provided to the liquefaction separation equipment LQ, the junction LBJ1 can be provided to any of the line LA, the line LC, and the second distillation column 20 to feed the extractant.

Although the line LB of the liquefaction separation equipment LQ comprises all of the junctions LBJ1 and LBJ2 and the condenser 26 as a liquefaction section in the second, fifth, and seventh embodiments, the line LB may comprise any one of the three or a combination of any two of the three. Of the junctions LBJ1 and LBJ2 and the condenser 26, a member not provided to the line LB can be provided to another member of the liquefaction separation equipment LQ, that is, the three-phase separator 23 or the gas-liquid separator 22. The second, fifth, and seventh embodiments are implementable even in the case that the line LB of the liquefaction separation equipment LQ comprises none of the junctions LBJ1 and LBJ2 and the condenser 26 but that the junction(s) LBJ1 and/or LBJ2 and/or condenser 26 are/is provided to another member of the liquefaction separation equipment LQ, that is, the three-phase separator 23 or the gas-liquid separator 22. In the case that the junction LBJ1 is not provided to the liquefaction separation equipment LQ, the junction LBJ1 can be provided to any of the line LA, the line LC, and the second distillation column 20 to feed the extractant.

Although the line LB of the liquefaction separation equipment LQ comprises the condenser 26 and the junction LBJ1 in the third and sixth embodiments, the line LB may comprise only one of them or none of them. Even when none of the condenser 26 and the junction LBJ1 is provided to the line LB, the fifth distillation column 50 can exert the function of liquefaction. In the case that the junction LBJ1 is not provided to the line LB, the junction LBJ1 can be provided to another member of the liquefaction separation equipment LQ such as the fifth distillation column 50. In the case that the junction LBJ1 is not provided to the liquefaction separation equipment LQ, the junction LBJ1 can be provided to any of the line LA, the line LC, and the second distillation column 20 to feed the extractant.

In the case that the condenser 26 and the junction LBJ1 are provided to the line LB in the above embodiments, any one of them may be provided in the upstream side in the line LB (the column top outlet 20t side of the second distillation column), though it is preferable for the cost of equipment that the condenser 26 be provided in the upstream side.

In the case that the junction LBJ2 is provided to the line LB in the above embodiments, the junction LBJ2 may be provided in the upstream side or downstream side of the condenser 26, and may be in the upstream side or downstream side of the junction LBJ1, though it is preferable for achievement of sufficient liquefaction that the junction LBJ2 be provided in the upstream side of the condenser 26 and junction LBJ1.

### (Connection partner of line LE)

In the case that the liquefaction separation equipment LQ does not comprise the junction LBJ1 in the above embodiments, the line LE may be connected to the line LA, the line LC, or the second distillation column 20 to feed the extractant from the source of extractant supply thereto, and even in the case that the liquefaction separation equipment LQ comprises the junction LBJ1, the flow (FS) of the extractant may be fed to the line LA, the line LC, or the second distillation column 20, for example, with the line LE2 branching from the line LE.

In the fourth to sixth embodiments, the flow (FSS) to be fed via the line LG may be fed, not to the third distillation column 30, but to at least one selected from the group consisting of the second distillation column 20, the liquefaction separation equipment LQ (i.e., the gas-liquid separator 22, the three-phase separator 23, the fifth distillation column 50, and the line LB), the line LC, and the line LA.

Although the line LF branches from the line LE in the fourth to sixth embodiments, the line LF may be connected to the column bottom outlet 30b of the third distillation column.

### REFERENCE SIGNS LIST

10i: inlet, 10b: column bottom outlet, 10t: column top outlet, 10: first distillation column, 20i: inlet, 20b: column bottom outlet, 20t: column top outlet, 20: second distillation column, 30i: first inlet, 30b: column bottom outlet, 30t: column top outlet, 30: third distillation column, LA, LB, LC, LD, LE, LF: line, 22: gas-liquid separator, 22g: gas phase outlet, 22o: liquid phase outlet, 22i: fluid inlet, 26: condenser (liquefaction section), LBJ1: junction (liquefaction section), LBJ2: junction (liquefaction section), 23: three-phase separator, 23g: gas phase outlet, 23o: oil phase outlet, 23i: fluid inlet, 23w: aqueous phase outlet, 24: oil-water separator, 24o: oil phase outlet, 24w: aqueous phase outlet, 40i: inlet, 40b: column bottom outlet, 40t: column top outlet, 40: fourth distillation column, 50i: inlet, 50b: column bottom outlet, 50t: column top outlet, 50: fifth distillation column, LQ: liquefaction separation equipment, 100, 110, 120, 200, 210, 220, 300: propylene oxide purification system.

## Claims

1. A propylene oxide purification system comprising:
a first distillation column comprising an inlet configured to receive crude propylene oxide, a column bottom outlet and a column top outlet;
a second distillation column comprising an inlet, a reflux inlet, a column bottom outlet and a column top outlet;
liquefaction separation equipment configured to liquefy at least part of a flow fed from the column top outlet of the second distillation column, and discharge a gas phase from a gas phase outlet and a liquid phase from a liquid phase outlet;
a third distillation column comprising an inlet, a column bottom outlet and a column top outlet;
a line LA connecting the column top outlet of the first distillation column and the inlet of the second distillation column;
a line LC connecting the liquid phase outlet of the liquefaction separation equipment and the reflux inlet of the second distillation column;
a line LD connecting the column bottom outlet of the second distillation column and the inlet of the third distillation column; and
a line LE connecting a source of extractant supply and at least one selected from the group consisting of the line LA, the liquefaction separation equipment, the line LC and the second distillation column.

2. The propylene oxide purification system according to claim 1, wherein the liquefaction separation equipment comprises: a gas-liquid separator comprising the gas phase outlet, the liquid phase outlet and a fluid inlet; a line LB connecting the column top outlet of the second distillation column and the fluid inlet of the gas-liquid separator; and a liquefaction section provided to the line LB or the gas-liquid separator.

3. The propylene oxide purification system according to claim 2, wherein the liquefaction section comprises a condenser.

4. The propylene oxide purification system according to claim 2 or 3, wherein the liquefaction section comprises a junction connecting the line LE and the line LB or the gas-liquid separator.

5. The propylene oxide purification system according to any one of claims 2 to 4, wherein the liquefaction section comprises a junction connecting a line LH connected to a source of water supply and the line LB or the gas-liquid separator, and satisfies (a) or (b):
(a) the gas-liquid separator is a three-phase separator further comprising an aqueous phase outlet;
(b) an oil-water separator comprising a mixture inlet, an oil phase outlet and an aqueous phase outlet is provided to the line LC, the mixture inlet is connected to the liquid phase outlet of the gas-liquid separator, and the oil phase outlet is connected to the reflux inlet of the second distillation column.

6. The propylene oxide purification system according to claim 1, wherein the liquefaction separation equipment comprises: a fifth distillation column comprising an inlet, the gas phase outlet provided to a column top of the fifth distillation column, and the liquid phase outlet provided to a column bottom of the fifth distillation column; and a line LB connecting the inlet of the fifth distillation column and the column top outlet of the second distillation column.

7. The propylene oxide purification system according to claim 6, wherein the liquefaction separation equipment further comprises a junction connecting the line LE and the line LB.

8. The propylene oxide purification system according to claim 6 or 7, wherein the liquefaction separation equipment further comprises a condenser provided to the line LB.

9. The propylene oxide purification system according to any one of claims 1 to 8, further comprising a line LJ connecting a detoxification apparatus and the gas phase outlet of the liquefaction separation equipment.

10. The propylene oxide purification system according to any one of claims 1 to 9, wherein the source of extractant supply is the column bottom outlet of the third distillation column.

11. The propylene oxide purification system according to any one of claims 1 to 10, further comprising:
a fourth distillation column comprising an inlet, a column bottom outlet and a column top outlet;
a line LF connecting the column bottom outlet of the third distillation column or a line connected to the column bottom outlet of the third distillation column and the inlet of the fourth distillation column; and
a line LG connecting the column bottom outlet of the fourth distillation column and at least one selected from the group consisting of the second distillation column, the liquefaction separation equipment, the line LC, the line LA and the third distillation column.

12. A method for producing purified propylene oxide with use of the propylene oxide purification system according to any one of claims 1 to 11, comprising:
a step of distilling crude propylene oxide in the first distillation column, to discharge a high-boiling-point component having a boiling point higher than that of propylene oxide from a column bottom of the first distillation column and a propylene oxide-containing flow (F1) removed of the high-boiling-point component from a column top of the first distillation column;
a step of feeding an extractant to at least one selected from the group consisting of the line LA, the liquefaction separation equipment, the line LC and the second distillation column;
a step of distilling the flow (F1) together with the extractant in the second distillation column, to discharge a flow (F2) comprising a C1-C4 hydrocarbon and propylene oxide from a column top of the second distillation column and a flow (F3) comprising the extractant and propylene oxide from a column bottom of the second distillation column;
a step of liquefying at least part of the propylene oxide contained in the flow (F2) in the liquefaction separation equipment, and discharging a gas phase flow (F2G) from the gas phase outlet and a liquid phase flow (F2O) from the liquid phase outlet;
a step of returning the liquid phase flow (F2O) to the second distillation column via the line LC; and
a step of distilling the flow (F3) in the third distillation column, to discharge a flow comprising the extractant from a column bottom of the third distillation column and purified propylene oxide from a column top of the third distillation column.

## Patentansprüche

1. Propylenoxidreinigungssystem, das umfasst:
eine erste Destillationskolonne mit einem Eingang, der so konfiguriert ist, dass er Rohpropylenoxid aufnimmt, einem Kolonnensumpfausgang und einem Kolonnenkopfausgang;
eine zweite Destillationskolonne mit einem Eingang, einem Rückflusseingang, einem Kolonnensumpfausgang und einem Kolonnenkopfausgang;
eine Verflüssigungsseparationsvorrichtung, die so konfiguriert ist, dass sie wenigstens einen Teil eines Stroms verflüssigt, der aus dem Kolonnenkopfausgang der zweiten Destillationskolonne zugeführt wird, und eine Gasphase aus einem Gasphasenausgang und eine Flüssigphase aus einem Flüssigphasenausgang abführt;
eine dritte Destillationskolonne mit einem Eingang, einem Kolonnensumpfausgang und einem Kolonnenkopfausgang;
eine Leitung LA, die den Kolonnenkopfausgang der ersten Destillationskolonne und den Eingang der zweiten Destillationskolonne verbindet;
eine Leitung LC, die den Flüssigphasenausgang der Verflüssigungsseparationsvorrichtung und den Rückflusseingang der zweiten Destillationskolonne verbindet;
eine Leitung LD, die den Kolonnensumpfausgang der zweiten Destillationskolonne und den Eingang der dritten Destillationskolonne verbindet; und
eine Leitung LE, die eine Extraktionsmittelzufuhrquelle und wenigstens eine ausgewählt aus der Gruppe bestehend aus der Leitung LA, der Verflüssigungsseparationsvorrichtung, der Leitung LC und der zweiten Destillationskolonne verbindet.

2. Propylenoxidreinigungssystem gemäß Anspruch 1, wobei die Verflüssigungsseparationsvorrichtung umfasst: einen Gas-Flüssigkeits-Separator mit dem Gasphasenausgang, dem Flüssigphasenausgang und einem Fluideingang; eine Leitung LB, die den Kolonnenkopfausgang der zweiten Destillationskolonne und den Fluideingang des Gas-Flüssigkeits-Separators verbindet; und einen Verflüssigungsbereich, der in der Leitung LB oder dem Gas-Flüssigkeits-Separator vorgesehen ist.

3. Propylenoxidreinigungssystem gemäß Anspruch 2, wobei der Verflüssigungsbereich einen Kondensator umfasst.

4. Propylenoxidreinigungssystem gemäß Anspruch 2 oder 3, wobei der Verflüssigungsbereich eine Verbindungsstelle umfasst, welche die Leitung LE und die Leitung LB oder den Gas-Flüssigkeits-Separator verbindet.

5. Propylenoxidreinigungssystem gemäß einem der Ansprüche 2 bis 4, wobei der Verflüssigungsbereich eine Verbindungsstelle umfasst, die eine an eine Wasserzufuhrquelle angeschlossene Leitung LH und die Leitung LB oder den Gas-Flüssigkeits-Separator verbindet und (a) oder (b) erfüllt:
(a) der Gas-Flüssigkeits-Separator ist ein Dreiphasen-Separator mit des Weiteren einem Ausgang für eine wässrige Phase;
(b) ein Öl-Wasser-Separator mit einem Mischungseingang, einem Ölphasenausgang und einem Ausgang für eine wässrige Phase ist in der Leitung LC vorgesehen, wobei der Mischungseingang mit dem Flüssigphasenausgang des Gas-Flüssigkeits-Separators verbunden ist und der Ölphasenaugang mit dem Rückflusseingang der zweiten Destillationskolonne verbunden ist.

6. Propylenoxidreinigungssystem gemäß Anspruch 1, wobei die Verflüssigungsseparationsvorrichtung umfasst: eine fünfte Destillationskolonne mit einem Eingang, wobei der Gasphasenausgang an einem Kolonnenkopf der fünften Destillationskolonne vorgesehen ist und der Flüssigphasenausgang an einem Kolonnensumpf der fünften Destillationskolonne vorgesehen ist; und einer Leitung LB, die den Eingang der fünften Destillationskolonne und den Kolonnenkopfausgang der zweiten Destillationskolonne verbindet.

7. Propylenoxidreinigungssystem gemäß Anspruch 6, wobei die Verflüssigungsseparationsvorrichtung des Weiteren eine Verbindungsstelle umfasst, welche die Leitung LE und die Leitung LB verbindet.

8. Propylenoxidreinigungssystem gemäß Anspruch 6 oder 7, wobei die Verflüssigungsseparationsvorrichtung des Weiteren einen Kondensator umfasst, der in der Leitung LB vorgesehen ist.

9. Propylenoxidreinigungssystem gemäß einem der Ansprüche 1 bis 8, das des Weiteren eine Leitung LJ umfasst, die eine Entgiftungsvorrichtung und den Gasphasenausgang der Verflüssigungsseparationsvorrichtung verbindet.

10. Propylenoxidreinigungssystem gemäß einem der Ansprüche 1 bis 9, wobei die Extraktionsmittelzufuhrquelle der Kolonnensumpfausgang der dritten Destillationskolonne ist.

11. Propylenoxidreinigungssystem gemäß einem der Ansprüche 1 bis 10, das des Weiteren umfasst:
eine vierte Destillationskolonne mit einem Eingang, einem Kolonnensumpfausgang und einem Kolonnenkopfausgang;
eine Leitung LF, die den Kolonnensumpfausgang der dritten Destillationskolonne oder eine mit dem Kolonnensumpfausgang der dritten Destillationskolonne verbundene Leitung mit dem Eingang der vierten Destillationskolonne verbindet; und
eine Leitung LG, die den Kolonnensumpfausgang der vierten Destillationskolonne mit mindestens einer aus der Gruppe bestehend aus der zweiten Destillationskolonne, der Verflüssigungsseparationsvorrichtung, der Leitung LC, der Leitung LA und der dritten Destillationskolonne verbindet.

12. Verfahren zur Herstellung von gereinigtem Propylenoxid unter Verwendung des Propylenoxidreinigungssystems nach einem der Ansprüche 1 bis 11, das umfasst:
einen Schritt des Destillierens von Rohpropylenoxid in der ersten Destillationskolonne, um einen hochsiedenden Bestandteil mit einem höheren Siedepunkt als Propylenoxid aus einem Kolonnensumpf der ersten Destillationskolonne und einen Propylenoxid enthaltenden Strom (F1), der von der hochsiedenden Komponente entfernt wurde, aus einem Kolonnenkopf der ersten Destillationskolonne abzuführen;
einen Schritt des Zuführens eines Extraktionsmittels zu wenigstens einer ausgewählt aus der Gruppe bestehend aus der Leitung LA, der Verflüssigungsseparationsvorrichtung, der Leitung LC und der zweiten Destillationskolonne;
eines Schritts des Destillierens des Stroms (F1) zusammen mit dem Extraktionsmittel in der zweiten Destillationskolonne, um einen Strom (F2), der C1-C4-Kohlenwasserstoff und Propylenoxid umfasst, aus einem Kolonnenkopf der zweiten Destillationskolonne und einen Strom (F3), der das Extraktionsmittel und Propylenoxid umfasst, aus einem Kolonnensumpf der zweiten Destillationskolonne abzuführen;
einen Schritt des Verflüssigens wenigstens eines Teils des in dem Strom (F2) enthaltenen Propylenoxids in der Verflüssigungsseparationsvorrichtung und des Abführens eines Gasphasenstroms (F2G) aus dem Gasphasenausgang und eines Flüssigphasenstroms (F2O) aus dem Flüssigphasenausgang;
einen Schritt des Rückführens des Flüssigphasenstroms (F2O) zur zweiten Destillationskolonne über die Leitung LC; und
einen Schritt des Destillierens des Stroms (F3) in der dritten Destillationskolonne, um einen Strom, der das Extraktionsmittel umfasst, aus einem Kolonnensumpf der dritten Destillationskolonne und gereinigtes Propylenoxid aus einem Kolonnenkopf der dritten Destillationskolonne abzuführen.

## Revendications

1. Système de purification d'oxyde de propylène comprenant :
une première colonne de distillation comprenant une entrée configurée pour recevoir un oxyde de propylène brut, une sortie de bas de colonne et une sortie de sommet de colonne ;
une deuxième colonne de distillation comprenant une entrée, une entrée de reflux, une sortie de bas de colonne et une sortie de sommet de colonne ;
un équipement de séparation par liquéfaction configuré pour liquéfier au moins une partie d'un écoulement introduit à partir de la sortie de sommet de colonne de la deuxième colonne de distillation, et évacuer une phase gazeuse à partir d'une sortie de phase gazeuse et une phase liquide à partir d'une sortie de phase liquide ;
une troisième colonne de distillation comprenant une entrée, une sortie de bas de colonne et une sortie de sommet de colonne ;
une conduite LA raccordant la sortie de sommet de colonne de la première colonne de distillation et l'entrée de la deuxième colonne de distillation ;
une conduite LC raccordant la sortie de phase liquide de l'équipement de séparation par liquéfaction et l'entrée de reflux de la deuxième colonne de distillation ;
une conduite LD raccordant la sortie de bas de colonne de la deuxième colonne de distillation et l'entrée de la troisième colonne de distillation ; et
une conduite LE raccordant une source d'alimentation en agent d'extraction et au moins un sélectionné dans le groupe consistant en la conduite LA, l'équipement de séparation par liquéfaction, la conduite LC et la deuxième colonne de distillation.

2. Système de purification d'oxyde de propylène selon la revendication 1, dans lequel l'équipement de séparation par liquéfaction comprend : un séparateur gaz-liquide comprenant la sortie de phase gazeuse, la sortie de phase liquide et une entrée de fluide ; une conduite LB raccordant la sortie de sommet de colonne de la deuxième colonne de distillation et l'entrée de fluide du séparateur gaz-liquide ; et une section de liquéfaction fournie à la conduite LB ou au séparateur gaz-liquide.

3. Système de purification d'oxyde de propylène selon la revendication 2, dans lequel la section de liquéfaction comprend un condenseur.

4. Système de purification d'oxyde de propylène selon la revendication 2 ou 3, dans lequel la section de liquéfaction comprend une jonction raccordant la conduite LE et la conduite LB ou le séparateur gaz-liquide.

5. Système de purification d'oxyde de propylène selon l'une quelconque des revendications 2 à 4, dans lequel la section de liquéfaction comprend une jonction raccordant une conduite LH raccordée à une source d'alimentation en eau et la conduite LB ou le séparateur gaz-liquide, et satisfait à (a) ou (b) :
(a) le séparateur gaz-liquide est un séparateur triphasique comprenant en outre une sortie de phase aqueuse ;
(b) un séparateur huile-eau comprenant une entrée de mélange, une sortie de phase huileuse et une sortie de phase aqueuse est fourni à la conduite LC, l'entrée de mélange est raccordée à la sortie de phase liquide du séparateur gaz-liquide, et la sortie de phase huileuse est raccordée à l'entrée de reflux de la deuxième colonne de distillation.

6. Système de purification d'oxyde de propylène selon la revendication 1, dans lequel l'équipement de séparation par liquéfaction comprend : une cinquième colonne de distillation comprenant une entrée, la sortie de phase gazeuse fournie à un sommet de colonne de la cinquième colonne de distillation, et la sortie de phase liquide fournie à un bas de colonne de la cinquième colonne de distillation ; et une conduite LB raccordant l'entrée de la cinquième colonne de distillation et la sortie de sommet de colonne de la deuxième colonne de distillation.

7. Système de purification d'oxyde de propylène selon la revendication 6, dans lequel l'équipement de séparation par liquéfaction comprend en outre une jonction raccordant la conduite LE et la conduite LB.

8. Système de purification d'oxyde de propylène selon la revendication 6 ou 7, dans lequel l'équipement de séparation par liquéfaction comprend en outre un condenseur fourni à la conduite LB.

9. Système de purification d'oxyde de propylène selon l'une quelconque des revendications 1 à 8, comprenant en outre une conduite LJ raccordant un appareil de détoxification et la sortie de phase gazeuse de l'équipement de séparation par liquéfaction.

10. Système de purification d'oxyde de propylène selon l'une quelconque des revendications 1 à 9, dans lequel la source d'alimentation en agent d'extraction est la sortie de bas de colonne de la troisième colonne de distillation.

11. Système de purification d'oxyde de propylène selon l'une quelconque des revendications 1 à 10, comprenant en outre :
une quatrième colonne de distillation comprenant une entrée, une sortie de bas de colonne et une sortie de sommet de colonne ;
une conduite LF raccordant la sortie de bas de colonne de la troisième colonne de distillation ou une conduite raccordée à la sortie de bas de colonne de la troisième colonne de distillation et l'entrée de la quatrième colonne de distillation ; et
une conduite LG raccordant la sortie de bas de colonne de la quatrième colonne de distillation et au moins un sélectionné dans le groupe consistant en la deuxième colonne de distillation, l'équipement de séparation par liquéfaction, la conduite LC, la conduite LA et la troisième colonne de distillation.

12. Procédé de production d'oxyde de propylène purifié avec utilisation du système de purification d'oxyde de propylène selon l'une quelconque des revendications 1 à 11, comprenant :
une étape de distillation d'oxyde de propylène brut dans la première colonne de distillation, pour évacuer un composant à point d'ébullition élevé présentant un point d'ébullition supérieur à celui de l'oxyde de propylène à partir d'un bas de colonne de la première colonne de distillation et un écoulement contenant de l'oxyde de propylène (F1) retiré du composant à point d'ébullition élevé à partir d'un sommet de colonne de la première colonne de distillation ;
une étape d'introduction d'un agent d'extraction dans au moins un sélectionné dans le groupe consistant en la conduite LA, l'équipement de séparation par liquéfaction, la conduite LC et la deuxième colonne de distillation ;
une étape de distillation de l'écoulement (F1) conjointement avec l'agent d'extraction dans la deuxième colonne de distillation, pour évacuer un écoulement (F2) comprenant un hydrocarbure en C1-C4 et de l'oxyde de propylène à partir d'un somment de colonne de la deuxième colonne de distillation et un écoulement (F3) comprenant l'agent d'extraction et l'oxyde de propylène à partir d'un bas de colonne de la deuxième colonne de distillation ;
une étape de liquéfaction d'au moins une partie de l'oxyde de propylène contenu dans l'écoulement (F2) dans l'équipement de séparation par liquéfaction, et d'évacuation d'un écoulement en phase gazeuse (F2G) à partir de la sortie de phase gazeuse et d'un écoulement en phase liquide (F2O) à partir de la sortie de phase liquide ;
une étape de renvoi de l'écoulement en phase liquide (F2O) vers la deuxième colonne de distillation via la conduite LC ; et
une étape de distillation de l'écoulement (F3) dans la troisième colonne de distillation, pour évacuer un écoulement comprenant l'agent d'extraction à partir d'un bas de colonne de la troisième colonne de distillation et de l'oxyde de propylène purifié à partir d'un sommet de colonne de la troisième colonne de distillation.
